# EUROPEAN PATENT APPLICATION

(11) **EP 3 760 055 A1**
(43) Date of publication of application: **06.01.2021**
(21) Application number: 19791742.0
(22) Date of filing: 04.04.2019
(51) Int. Cl.: A23L 33/135, A23K 10/16, A23K 20/163, A23L 33/125, A61K 31/715, A61K 35/747, A61K 36/60, A61P 1/16, A61P 3/04, A61P 3/06, A61K 127/00

(54) **COMPOSITION FOR INHIBITING FAT ACCUMULATION**

(30) Priority: 25.04.2018 JP 2018084048
(71) Applicant: Sone Farm Co., Ltd., Tokyo 160-0022 (JP)
(72) Inventor: SUGIYAMA,Masanori, Hiroshima-shi, Hiroshima 732-0063 (JP)
(74) Representative: Papula Oy
(86) International application number: PCT/JP2019/014997
(87) International publication number: WO 2019/208150

(57) **Abstract**

A composition comprises, as an active ingredient, a cell body of a lactic acid bacterium belonging to *Lactobacillus paracasei,* a cultured product or fermented product thereof, or a polysaccharide produced thereby is effective in suppressing fat accumulation and can be used as a food or drink, a medicine, and a feed.

## Description

### Technical Field

The present invention relates to a composition for suppressing fat accumulation. More specifically, the present invention relates to a composition for suppressing fat accumulation, comprising, as an active ingredient, a cell body of a lactic acid bacterium belonging to *Lactobacillus paracasei,* a cultured product or fermented product thereof, or a polysaccharide produced thereby.

### Background Art

In recent years, the number of patients with a clinical condition called a metabolic syndrome, which shows symptoms such as fatty liver, diabetes, hypertension, and arteriosclerosis, has increased in Japan, Europe, and the United States. Obesity is considered to be deeply involved in a metabolic syndrome. Obesity refers to a state in which the number of fat cells increases or fat is excessively accumulated in fat cells, by an excessive intake of energy due to overeating or a decrease in energy consumption due to insufficient exercise. It is therefore considered that obesity is prevented and improved by suppressing such fat accumulation.

In addition, fatty liver refers to a state in which neutral fats are excessively accumulated in liver or hepatocytes and is considered to be closely related to lifestyle-related diseases such as obesity, hyperlipidemia, and diabetes. It is considered as one of causes for accumulation of neutral fats in liver or hepatocytes that free fatty acids are taken up in liver or hepatocytes and accumulated therein as neutral fats.

Various suggestions, such as ingestion of foods or supplements that promote the burning of body fat or suppress fat accumulation, and administration of an anti-obesity agent in the medical field, have been proposed to prevent and improve obesity. In addition, a method for suppressing fat accumulation in liver and the like have been proposed as a method for preventing and treating fatty liver. At present, however, from the viewpoint of safety and practicality, there are only a few that effectively prevent and improve obesity and fatty liver.

On the other hand, lactic acid bacteria have been used for a long period of time when producing food products such as dairy products including fermented milk, lactic acid bacteria beverages, and fermented butter, and also utilized as materials for medicines, pharmaceuticals and the like because of their own effect on the regulation of intestine functions and their own various pharmacological actions. It has been reported as the action of lactic acid bacteria on fat accumulation that bacterial strains having a visceral fat-reducing effect were found among lactic acid bacteria of the genus *Lactobacillus* and *Lactococcus* (Patent Document 1); that lactic acid bacteria belonging to *Lactobacillus gasseri* have a fatty liver-inhibitory effect (Patent Document 2); and the like. In addition, as plant-derived lactic acid bacteria, *Pediococcus pentosaceus* strain LP28 and the like are known which have an effect of improving fatty liver and suppressing body fat accumulation (Non-Patent Document 3).

### Prior Art Documents

### Patent Documents

Patent Document 1: JP 2008-214253 A
Patent Document 2: JP 2008-24680 A

### Non-Patent Documents

Non-Patent Document 1: Appl. Microbiol. Biotechnol., 84, 341-347 (2009)

### Summary of Invention

### Problem to be solved by invention

Under such background arts, it has been desired to develop a new composition for suppressing fat accumulation, containing a lactic acid bacterium as an active ingredient. The problem to be solved by the present invention is thus to provide a new composition for suppressing fat accumulation, comprising a lactic acid bacterium as an active ingredient, which is effective in preventing or improving obesity, fatty liver and liver dysfunction.

### Means for solving the problem

The present inventor has made conducted intensive studies with the aim of developing a new composition for suppressing fat accumulation. As a result, the present inventor has found that polysaccharides produced by lactic acid bacteria belonging to *Lactobacillus paracasei* suppress hepatocytes from intaking free fatty acids to accumulate them as neutral fats; and that lactic acid bacteria belonging to *Lactobacillus paracasei* suppress body weight gain and visceral fat accumulation to have an anti-obesity action. On the basis of these findings, the present inventor has further studied and completed the present invention.

In one aspect of the present invention, the present invention relates to a composition for suppressing fat accumulation, comprising, as an active ingredient, a cell body of a lactic acid bacterium belonging to *Lactobacillus paracasei,* a cultured product or fermented product thereof, or a polysaccharide produced thereby.

The composition of the present invention can be preferably used to prevent or improve obesity and also prevent or improve fatty liver or liver dysfunction.

In the composition of the present invention, the lactic acid bacterium is preferably a lactic acid bacterium derived from a fig, and particularly preferably *Lactobacillus paracasei* strain IJH-SONE68 (Accession No. NITE BP-02242) or a lactic acid bacterium equivalent thereto.

In the composition of the present invention, a cultured product or fermented product of the lactic acid bacterium is preferably the one that can be obtained by cultivating or fermenting the lactic acid bacteria in the presence of a fruit juice of pineapple genus plant.

In the composition of the present invention, a polysaccharide produced by the lactic acid bacterium includes a neutral polysaccharide having a structure in which N-acetylglucosamines are linked with each other via α-1,6 bond, and an acidic polysaccharide composed mainly of glucoses and mannoses.

The composition of the present invention is preferably a food or drink composition, and the food or drink include a beverage, a functional food, a fermented food, and a supplement.

In addition, the composition of the present invention is preferably a pharmaceutical composition.

Furthermore, the composition of the present invention is preferably a feed composition.

### Effect of Invention

The composition of the present invention is effective in suppressing fat accumulation and can be used to prevent or improve obesity and also prevent or improve fatty liver or liver dysfunction. The composition of the present invention can be used as a food or drink, a medicine, and a feed. The composition of the present invention comprises, as an active ingredient, a cell body of a lactic acid bacterium belonging to *Lactobacillus paracasei,* a cultured product or fermented product thereof, or a polysaccharide produced thereby. Therefore, the composition of the present invention has high safety, can be applied for a long period of time, can be supplied in a large amount at low cost, and has extremely high utility and practicality.

### Brief Description of Drawings

[Fig. 1] Fig. 1 shows microscope photographs of *Lactobacillus paracasei* strain IJH-SONE68. (A) in Fig. 1 is a gram-stained microscope photograph, and (B) in Fig. 1 is a scanning electron microscope (SEM) photograph.
[Fig. 2] Fig. 2 illustrates an isolation profile of anion exchange chromatography (TOYOPEARL DEAE-650M resin (Tosoh Corporation)) of exopolysaccharides from *Lactobacillus paracasei* strain IJH-SONE68. The exopolysaccharides were eluted with NaCl having a gradient concentration of 0 mM to 500 mM (broken line), and the exopolysaccharides in each fraction were monitored at 490 nm by a phenol sulfuric acid method (straight line).
[Fig. 3] Fig. 3 illustrates each NMR profile obtained by subjecting a neutral exopolysaccharide, which was obtained by purifying exopolysaccharides from *Lactobacillus paracasei* strain IJH-SONE68 with anion exchange column chromatography, to proton-NMR and carbon-NMR. (A) in Fig. 3 is the NMR profile of proton-NMR, and (B) in Fig. 3 is the NMR profile of carbon-NMR.
[Fig. 4] Fig. 4 illustrates results of structurally analyzing a neutral exopolysaccharide on the basis of the NMR profiles. These structural analysis results revealed that the neutral exopolysaccharide of *Lactobacillus paracasei* strain IJH-SONE68 has a structure in which N-acetylglucosamines are linked with each other via α-1,6 bond.
[Fig. 5] Fig. 5 is a graph illustrating that polysaccharides produced by *Lactobacillus paracasei* strain IJH-SONE68 has an action of suppressing fat accumulation in a concentration-dependent manner.
[Fig. 6] Fig. 6 is a graph illustrating changes in body weight gain of obesity model mice ingesting high fat diets, which ingested a fermented pineapple juice fermented with *Lactobacillus paracasei* strain IJH-SONE68.
[Fig. 7] Fig. 7 is a graph illustrating that the body weight gain of obesity model mice ingesting high fat diets was suppressed by allowing the mice to ingest a fermented pineapple juice fermented with *Lactobacillus paracasei* strain IJH-SONE68.
[Fig. 8] Fig. 8 is a graph illustrating that the visceral fat amount of obesity model mice ingesting high fat diets was suppressed by allowing the mice to ingest a fermented pineapple juice fermented with *Lactobacillus paracasei* strain IJH-SONE68.

### Embodiments for Carrying out Invention

The following detailed disclosures are made on the composition provided by the present invention for suppressing fat accumulation, comprising, as an active ingredient, a cell body of a lactic acid bacterium belonging to *Lactobacillus paracasei,* a cultured product or fermented product thereof, or a polysaccharide produced thereby.

### 1. The lactic acid bacterium in the present invention

The lactic acid bacterium in the present invention is a lactic acid bacterium belonging to *Lactobacillus paracasei* and preferably a lactic acid bacterium derived from a fig. Specifically, the lactic acid bacterium includes *Lactobacillus paracasei* strain IJH-SONE68 that was isolated and identified from leaves of a fig according to the present invention. This strain was nationally deposited under the accession number of NITE P-02242 at Patent Microorganisms Depositary, National Institute of Technology and Evaluation (#122, 2-5-8 Kazusakamatari, Kisarazu-shi, Chiba 292-0818, Japan) on April 19, 2016. The deposition was then transferred to an international deposit under the Budapest Treaty and given the international deposit accession number of NITE BP-02242 on May 26, 2017.

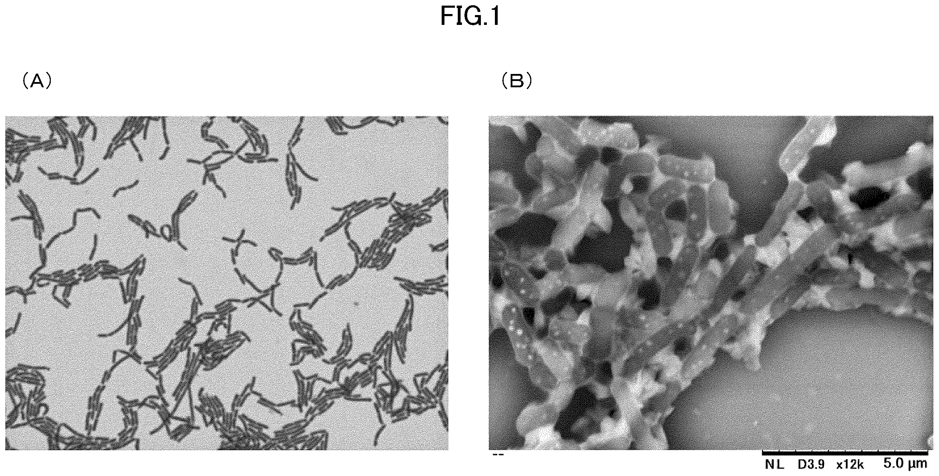

As illustrated in the photograph of Fig. 1, *Lactobacillus paracasei* strain IJH-SONE68 is a catalase-negative, gram-positive bacillus, and has mycological properties of forming a white colony and the mycological characteristic of conditional heterolactic fermentation. Furthermore, the strain has an ability to produce polysaccharides.

A lactic acid bacteria equivalent to *Lactobacillus paracasei* strain IJH-SONE68 is also included in the present invention. Here, the equivalent lactic acid bacterium indicates a lactic acid bacterium that belongs to *Lactobacillus paracasei* and has an action of suppressing fat accumulation, like *Lactobacillus paracasei* strain IJH-SONE68. In addition, the equivalent lactic acid bacterium also indicates a lactic acid bacterium that produces polysaccharides having an action of suppressing fat accumulation, like polysaccharides produced by *Lactobacillus paracasei* strain IJH-SONE68. These equivalent lactic acid bacteria are obtained, for example, by performing usual mutation treatment technique, such as mutation and genetic recombination, on *Lactobacillus paracasei* strain IJH-SONE68 and, in addition, may be bacterial strains that have been bred by selecting natural mutation strains of *Lactobacillus paracasei* strain IJH-SONE68, and the like.

### 2. The active ingredient in the present invention

The composition of the present invention contains, as an active ingredient, a cell body of the above-mentioned lactic acid bacterium, a cultured product or fermented product thereof, or a polysaccharide produced thereby.

The lactic acid bacterium can be cultured by a commonly used culture method such as liquid stationary culture, using a commonly used MRS medium or a modified medium thereof. The lactic acid bacterium can promote its growth by the culture in the presence of a fruit juice of a pineapple genus plant or its extract (WO 2011/046194 A). In addition, the lactic acid bacterium can also promote its growth by the culture in the presence of sake lees, sake lees extract or sake lees enzymes (JPH 03-172171 A, JPH 05-15366 A, and JP 2835548 B). As the lactic acid bacterium, a culture obtained after the cultivation may be used as it is, the obtained culture solution may be diluted or concentrated to be used, or the cell body recovered from the culture may be used. In addition, as long as the effect of the present invention is not impaired, various additional operations such as heat and freeze-dry may also be performed after the cultivation. The lactic acid bacterium of the present invention may be viable or dead and may include both viable and dead, while polysaccharides are adhered to the cell surface of the lactic acid bacterium. The dead lactic acid bacterium may be crushed and preferably has polysaccharides adhered to the cell surface thereof. In addition, the fermented product of the lactic acid bacterium can be obtained by fermenting the lactic acid bacterium usually using glucose or the like as a nutrient source, and further using yeast extract, fruit juice of pineapple genus plants, sake lees, distilled spirit lees, etc., if necessary.

Polysaccharides produced by the lactic acid bacterium can be obtained by isolating and purifying them from a culture of lactic acid bacteria belonging to *Lactobacillus paracasei* according to an ordinary method. Specifically, for example, the polysaccharides can be obtained by removing the cell body from a culture of lactic acid bacteria belonging to *Lactobacillus paracasei* by centrifugation, and precipitating polysaccharides from the obtained culture using ethanol, acetone, or the like. In addition, the polysaccharides can be obtained by further isolating and purifying them by ion exchange chromatography.

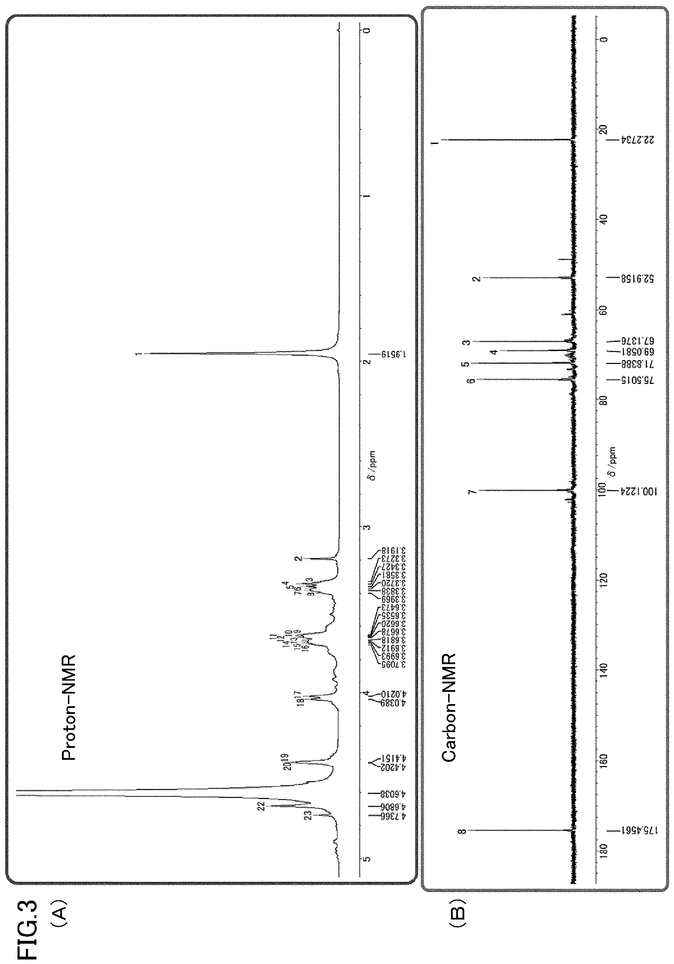

In the present invention, the polysaccharides produced by the lactic acid bacterium specifically include a neutral polysaccharide having a structure in which N-acetylglucosamines are linked with each other via α-1,6 bond, and an acidic polysaccharide composed mainly of glucoses and mannoses. This neutral polysaccharide can be obtained by isolating and purifying polysaccharides obtained from a culture of *Lactobacillus paracasei* strain IJH-SONE68 by anion exchange chromatography, as disclosed in Example 2 herein below. It was revealed from the proton-NMR and carbon-NMR profiles illustrated in Fig. 3 that this neutral polysaccharide has a structure in which N-acetylglucosamines are linked with each other by α-1,6 bonds. In addition, the *Lactobacillus paracasei* strain IJH-SONE68 secretes an acidic polysaccharide mainly composed of glucose and mannose outside the cell body. More specifically, this acidic polysaccharide is composed of glucose, mannose, galactose, and rhamnose, and their composition ratio is approximately 10:170: 2:1.

### 3. The composition in the present invention

The composition of the present invention can be used in various forms of a food or drink composition, a pharmaceutical composition, and a feed composition.

The food or drink of the food or drink composition are not particularly limited but include beverages such as soft drinks, carbonated drinks, nutritional drinks, fruit juice beverages, and lactic acid bacteria beverages, concentrated stock solutions of these beverages, powders for the preparation of these beverages, and the like; ice cream, sherbet and ice confectionery such as shaved ice; confectioneries such as candy, gummy, cereal, chewing gum, candy, gum, chocolate, tablet candy, snack, biscuit, jelly, jam, cream, and baked confectionery; dairy products such as processed milk, milk drink, fermented milk, drink yogurt, and butter; bread; enteral nutritious food, liquid food, childcare milk, sports drink; food such as puree; seat sake; and other functional foods. In addition, the food or drink may be supplements, and the supplements may be in the form of, for example, granules, powders, or tablets.

The food or drink as disclosed above may be prepared by adding a cell body of a lactic acid bacterium belonging to *Lactobacillus paracasei,* a cultured product or fermented product thereof, or a polysaccharide produced thereby, to raw materials of food or drink, or prepared in the same manner as an usual food or drink. The addition of a cell body of a lactic acid bacterium belonging to *Lactobacillus paracasei,* a cultured product or fermented product thereof, or a polysaccharide produced thereby may be performed at any stage of the process of preparing the food or drink. The food or drink may be prepared after a fermentation process of the added lactic acid bacteria. Examples of such food or drink include fermented foods such as lactic acid bacterium beverages and fermented milks.

The content of a cell body of the lactic acid bacterium or a cultured product or fermented product thereof in the food or drink composition may be appropriately determined depending on the embodiment of the food or drink, but is the one so that a cell body of the lactic acid bacterium is usually contained in the food or drink composition preferably in the range of 1×10⁶ to 1×10¹² cfu/g or 1×10⁶ to 1×10¹² cfu/ml, more preferably in the range of 1×10⁷ to 1×10¹¹ cfu/g or 1×10⁷ to 1×10¹¹ cfu/ml. In the case where the lactic acid bacterium is a dead cell body, the cfu/g or cfu/ml can be replaced with the number of cells per g or the number of cells per ml. In the case of the polysaccharide produced by the lactic acid bacterium, the polysaccharide is usually contained in the food or drink composition at an amount of 0.001% or more by weight, preferably at an amount of 0.01% or more by weight, in terms of the weight of the polysaccharide.

The pharmaceutical composition is usually used by blending a cell body of the lactic acid bacterium, a cultured product or fermented product thereof or a polysaccharide produced thereby with a physiologically acceptable liquid or solid of a pharmaceutical carrier usually used, followed by the formulation. The dosage form of the pharmaceutical composition is not particularly limited, but includes tablets, pills, powders, solutions, suspensions, emulsions, granules, capsules, syrups, suppositories, injections, ointments, patches, eye drops, and nose drops.

The content of a cell body of the lactic acid bacterium or a cultured product or fermented product thereof in the pharmaceutical composition may be appropriately determined depending on the dosage form, the dosage regimen, the age and sex of a subject, the kind of disease, the degree of disease, other conditions and the like, but is the one so that a cell body of the lactic acid bacterium is usually contained in the pharmaceutical composition preferably in the range of 1×10⁶ to 1×10¹² cfu/g or 1×10⁶ to 1×10¹² cfu/ml, more preferably in the range of 1×10⁷ to 1×10¹¹ cfu/g or 1×10⁷ to 1×10¹¹ cfu/ml. In the case where the lactic acid bacterium is a dead cell body, the cfu/g or cfu/ml can be replaced with the number of cells per g or the number of cells per ml. In the case of the polysaccharide produced by the lactic acid bacterium, the polysaccharide is usually contained in the pharmaceutical composition at an amount of 0.001% or more by weight, preferably at an amount of 0.01% or more by weight, in terms of the weight of the polysaccharide.

The administration timing of the pharmaceutical composition of the present invention is not particularly limited but may be appropriately chosen depending on a subject to be applied. The pharmaceutical composition may also be administered prophylactically or used in a maintenance therapy. The administration mode may be preferably appropriately determined depending on the dosage form, age, sex and other conditions of the administered subject, the degree of symptoms of the administered subject, and the like. In any case, the pharmaceutical composition of the present invention may be administered once per day, administered dividedly into two or more times or administered once every several days or weeks.

Examples of the feed of a feed composition include pet food, livestock feed and fish feed. Such a feed may be prepared by mixing common feed, for example, cereals, cakes, brans, fish meals, bone meals, oils and fats, skim milk powders, wheys, bitterns, mineral feeds, yeasts, or the like with a cell body of the lactic acid bacterium, a cultured product or fermented product thereof or a polysaccharide produced thereby. In addition, for example, like the case of silage, a feed may be prepared through a fermentation process with the lactic acid bacterium added thereto. The prepared feed may be orally administered to general mammals, livestock, farmed fishes, pet animals or the like. In the case of farmed fishes, it may be adopted to spread fermented products, to which a cell body of the lactic acid bacterium, a cultured product or fermented product thereof or a polysaccharide produced thereby is added, to the farmed place of fishes.

The content of a cell body of the lactic acid bacterium or a cultured product or fermented product thereof in the feed composition may be appropriately determined depending on the embodiment of the feed or the administered subject, but is the one so that a cell body of the lactic acid bacterium is usually contained in the feed composition preferably in the range of 1×10⁶ to 1×10¹² cfu/g or 1×10⁶ to 1×10¹² cfu/ml, more preferably in the range of 1×10⁷ to 1×10¹¹ cfu/g or 1×10⁷ to 1×10¹¹ cfu/ml. In the case where the lactic acid bacterium is a dead cell body, the cfu/g or cfu/ml can be replaced with the number of cells per g or the number of cells per ml. In the case of the polysaccharide produced by the lactic acid bacterium, the polysaccharide is usually contained in the feed composition at an amount of 0.001% or more by weight, preferably at an amount of 0.01% or more by weight, in terms of the weight of the polysaccharide.

### 4. Use of the composition of the present invention

A cell body of a lactic acid bacterium belonging to *Lactobacillus paracasei,* a cultured product or fermented product thereof, or a polysaccharide produced thereby has an action of suppressing hepatocytes from intaking free fatty acids to accumulate them as neutral fats, and suppresses body weight gain and visceral fat accumulation to have an anti-obesity action. Therefore, a composition containing, as an active ingredient, a cell body of a lactic acid bacterium belonging to *Lactobacillus paracasei,* a cultured product or fermented product thereof, or a polysaccharide produced thereby can be used to suppress fat accumulation and also suppress obesity. Specifically, the composition of the present invention can be used to prevent or improve obesity and also prevent or improve fatty liver or liver dysfunction. In addition, the composition of the present invention is effective in preventing or improving obesity and can be therefore utilized as materials of food or drink for maintaining and promoting health. In addition, the prevention or improvement of fatty liver or liver dysfunction leads to the maintenance, enhancement, recovery, and the like of physical strength, and the composition of the present invention can be therefore used as materials for food or drink for the maintenance, enhancement, and recovery of physical strength.

### Examples

Hereinafter, the present invention will be disclosed in more detail with reference to examples, but the present invention is not limited by these examples.

### Example 1

### Isolation and identification of lactic acid bacterium

### 1. Isolation of lactic acid bacterium sample

The leaves, stems, and fruits of a fig (variety "TOYOMITSU HIME") were chosen and cut into pieces of 2 to 3 mm using sterilized tweezers and scissors. Every five to six pieces were each then placed in a sterilized test tube containing MRS liquid medium, and statically cultured at 28°C and 37°C until the MRS medium as a standard medium for a lactic acid bacterium became turbid (proliferated). By the way, it took 2 to 4 days for the proliferation of the lactic acid bacterium candidate strains to be visible.

A part of each culture liquid of the lactic acid bacterium candidate strains was subjected to a line drawing paint on MRS agar medium using a disposable loop, followed by stationary culture. Among colonies formed on the agar medium, all of differently colored, lustrous and shaped colonies were picked up and subjected to a line drawing paint on a fresh MRS agar medium, and the colonies were purified.

H₂O₂ test was performed for each purified colony to verify the presence or absence of the production of a catalase enzyme. This is a test method for observing the presence or absence of oxygen generated when catalase is present, which is observed when microbial bodies are exposed to 10% H₂O₂ solution. By the way, a lactic acid bacterium produces no catalase.

As a result of attempting the search and isolation from a fig, one lactic acid bacterium candidate strain showing catalase-negative was obtained from the leaves of a fig as the isolation source.

### 2. Identification of the isolated strain

The aforementioned lactic acid bacterium candidate strain was again cultured in MRS liquid medium, and the microbial bodies were obtained by centrifugation. After the microbial bodies were treated with cell wall lytic enzyme, a genomic DNA was extracted using DNAzol reagent.

According to the method as disclosed in Lane, DJ (1991), "16S/23S rRNA sequencing", Nucleic Acid Techniques in Bacterial Systematics, pp. 115-175, edited by E. Stackebrandt & M. Goodfellow. Chichester: Wiley, a genomic DNA PCR was performed using a genomic DNA as a template and using 27f primer and 1525r primer, thereby to amplify 16S rDNA part. Then, an objective fragment was recovered from agarose gel according to NucleoSpin Gel and PCR Clean-up kit (manufactured by Mahalay Nagel). A sequencing reaction by a dye terminator method for sequencing a base sequence was performed with Big Dye Terminator Cycle Sequencing FS Ready Reaction Kit ver. 3.1 (manufactured by ThermoFisher Scientific), and analysis was made with ABI PRISM 3130xl Genetic Analyzer (manufactured by ThermoFisher Scientific). The base sequence of the analyzed 16S rDNA was subjected to a homology search by BLAST program and compared with the database of DNA data bank (DDBJ/EMBL/GenBank) to make a taxonomic identification on the isolated strain.

The lactic acid bacterium candidate strain isolated from leaves of a fig was named strain IJH-SONE68 and identified as *Lactobacillus paracasei* because it was 100% identical to a base sequence which was in the strain of *Lactobacillus paracasei* R094 already registered in DNA data bank (DDBJ/EMBL/GenBank) and which had NR-025880 as the accession number of the base sequence.

This strain was nationally deposited under the accession number of NITE P-02242 at Patent Microorganisms Depositary, National Institute of Technology and Evaluation (#122, 2-5-8 Kazusakamatari, Kisarazu-shi, Chiba 292-0818, Japan) on April 19, 2016. The deposition was then transferred to an international deposit under the Budapest Treaty and given the international deposit accession number of NITE BP-02242 on May 26, 2017.

### 3. Mycological properties of separated and identified lactic acid bacterium

The aforementioned isolated and identified lactic acid bacterium strain IJH-SONE68 was a catalase-negative, gram-positive rod and had a white colony forming property, as shown in the photograph of Fig. 1, and further had the characteristic of conditional hetero-lactic acid fermentation and the ability of producing polysaccharides.

### 4. Saccharide assimilation ability of the isolated and identified lactic acid bacterium

### (1). Test method of assimilation ability

The strain IJH-SONE68 was investigated for the assimilation ability of 49 kinds of saccharides according to the following test method.

The strain IJH-SONE68 was statically cultured in MRS liquid medium until the proliferation stationary phase. The microbial bodies obtained by centrifugation were washed with an appropriate amount of a suspension medium (manufactured by BioMeieux), and finally suspended in 2 mL of a suspension medium. A portion of the resultant suspension was added to 5 mL of a suspension medium to determine an amount (n) for McFarland turbidity to become 2. Subsequently, 2n of a microbial solution was added to API 50 CHL medium (manufactured by BioMerieux), and this solution was dispended to each well of API 50 CHL kit (manufactured by BioMerieux, 49 kinds of saccharides were coated on the bottom of each well). Finally, mineral oil was overlaid and set in a tray containing a sterilized water. After culturing at 37°C for 48 hours, the presence or absence of the assimilation ability was assessed by observing the change in color tone in each well.

### 5. Test results of the assimilation ability

Table 1 shows the results of investigating the assimilation ability of the strain IJH-SONE68 against 49 kinds of saccharides.

**[Table 1]**

| Assimilation abilities of the strain IJH-SONE68 against saccharides | |
|---|---|
| Substrates | Assimilation abilities |
| Control | - |
| Glycerol | - |
| Erythritol | - |
| D-Arabinose | - |
| L-Arabinose | - |
| D-Ribose | + |
| D-Xylose | - |
| L-Xylose | - |
| D-Adonitol | + |
| Methyl-βD-xylopyranoside | - |
| D-Galactose | + |
| D-Glucose | + |
| D-Fructose | + |
| D-Mannose | + |
| L-Sorbose | + |
| L-Rhamnose | - |
| Dulcitol | - |
| Inositol | - |
| D-Mannitol | + |
| D-Sorbitol | - |
| Methyl-αD-mannopyranoside | - |
| Methyl-αD-glucopyranoside | - |
| N-Acetylglucosamine | + |
| Amygdalin | - |
| Arbutin | - |
| Esculin + Ferric citrate | + |
| Salicin | + |
| D-Cellobiose | + |
| D-Maltose | + |
| D-Lactose | - |
| D-Melibiose | - |
| D-Sucrose | + |
| D - Trehalose | + |
| Inulin | - |
| D-Melezitose | + |
| D-Raffinose | - |
| Starch | - |
| Glycogen | - |
| Xylitol | - |
| Gentibiose | + |
| D-Turranose | - |
| D-Lyxose | - |
| D-Tagatose | + |
| D-Fucose | - |
| L-Fucose | - |
| D-Arabitol | - |
| L-Arabitol | - |
| Gluconic acid | + |
| 2-Ketogluconic acid | - |
| 5-Ketogluconic acid | - |

| | |
|---|---|
| In Table 1, + indicates the possession of assimilation ability, and - indicates no possession of assimilation ability. | |

### Example 2

### 1. Isolation and purification of exopolysaccharides produced by the strain IJH-SONE68

Exopolysaccharides produced by the strain IJH-SONE68 were isolated and purified according to the following method.

The strain IJH-SONE68 was statically cultured in MRS liquid medium until the proliferation stationary phase. 5 mL of the resultant culture solution was used as a seed culture solution, and inoculated on 5 L of a semisynthetic medium for producing exopolysaccharides (the composition thereof will be disclosed below), followed by static culture at 37°C for 120 hours. After the resultant culture solution was cooled to 4°C, proteins contained in the culture supernatant were denatured, and 202.5 mL of a 100% trichloroacetic acid aqueous solution was added thereto, mixed and allowed to stand for 30 minutes to remove them as precipitates in a later step. After the precipitates were removed by centrifugation, an equal amount of acetone was added to the collected supernatant and mixed, and the resultant mixture was allowed to stand at 4°C overnight to precipitate polysaccharides produced by the strain IJH-SONE68. The precipitates were collected by centrifugation, and the resultant precipitates were then washed with 250 mL of 70% ethanol. After the precipitates were air-dried, 75 mL of 50 mM Tris-HCl buffer (pH 8.0) was added to the resultant precipitates and mixed for 1 hour to dissolve the precipitates. After insoluble impurities were removed by centrifugation to recover a supernatant, 750 µL of 1 mg/mL DNase solution (Worthington, Inc.) and 750 µL of 1 mg/mL RNase solution (Nacalai Tesque, Inc.) were each added to the recovered supernatant, followed by being allowed to react at 37°C for 8 hours. Subsequently, 750 µL of 2 mg/mL proteinase K solution (manufactured by Wako Pure Chemical Industries, Ltd.) was added, and the resultant mixture was reacted at 37°C for 16 hours. The resultant solution after the reaction was cooled to 4°C, the added enzymes were each denatured, and 8.75 mL of a 100% trichloroacetic acid aqueous solution was then added thereto, mixed and allowed to stand for 1 hour to remove the enzymes as precipitates in the next centrifugation. The resultant precipitates were removed by centrifugation to obtain a supernatant, 262.5 mL of 100% ethanol was added to the obtained supernatant, the resultant mixture was thoroughly mixed, and the polysaccharides produced by the strain IJH-SONE68 strain were then recovered as precipitates by centrifugation. After the precipitates were washed with 50 mL of 70% ethanol, the precipitates were air-dried, an appropriate amount (about 25 mL) of a purified water was added thereto, and the resultant mixture was allowed to stand overnight at 4°C to dissolve the polysaccharides. For the polysaccharides sample after the dissolution, small molecules such as monosaccharides in the recovered sample were removed using an ultrafiltration unit (Merck Ltd.) of 10,000 MWCO while replacing the solvent with a purified water, and a purified polysaccharide sample was thus obtained.

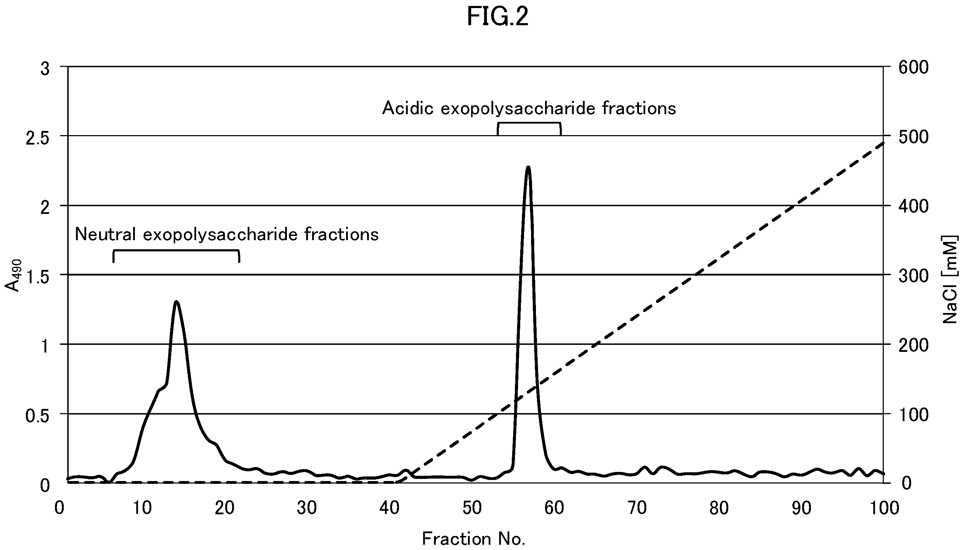

The purified polysaccharide sample was applied to an open column (2.5 × 22 cm) packed with TOYOPEARL DEAE-650M resin (Tosoh Corporation) previously equilibrated with 50 mM Tris-HCl buffer (pH 8.0), and column work was performed to isolate and purify the sample to neutral polysaccharide fractions and acidic polysaccharide fractions. The same buffer was used as an elution solution, and a flow rate was fixed at 1 mL/min. In addition, eluates were collected in different test tubes at every 6 mL. First, from the beginning to 240 minutes, elution was made with the same buffer (Test Tube Nos. 1 to 40). Next, from 240 minutes to 600 minutes, a concentration gradient of 0 to 500 mM NaCl was prepared using the same buffer, and elution was continued with the gradient (Test Tube Nos. 41 to 100). The column isolation spectrum is illustrated in Fig. 2. After the presence of polysaccharides was confirmed by a phenol sulfuric acid method (disclosed below) for all the samples eluted in the test tubes, the confirmed solutions in the test tubes were collected as neutral polysaccharide fractions and acidic polysaccharide fraction, respectively. For each fraction, an ultrafiltration unit of 10,000 MWCO was used to remove small molecules such as monosaccharides in the recovered sample while replacing the solvent with purified water.

As a result, neutral polysaccharide fractions and acidic polysaccharide fractions were isolated and purified as exopolysaccharides produced by the strain IJH-SONE68.

A semisynthetic medium for producing polysaccharides was prepared by modifying a medium disclosed in Kimmel SA, Roberts RF., "Development of a growth medium suitable for exopolysaccharide production by Lactobacillus delbrueckii ssp. Bulgaricus RR.", Int. J. Food Microbiol., 40, 87-92 (1998), as follows:

| Semisynthetic medium for producing polysaccharides [g/L] | |
|---|---|
| Glucose | 20 |
| Tween 80 | 1.0 |
| Ammonium citrate | 2.0 |
| Sodium acetate | 5.0 |
| MgSO₄·7H₂O | 0.1 |
| MnSO₄·5H₂O | 0.05 |
| K₂HPO₄ | 2.0 |
| Bacto casitone | 10.0 |
| Vitamin Soln. | 2 mL |
| Trace element Soln. | 1 mL |

| Vitamin Soln. | [g/L] |
|---|---|
| 4-Aminobenzoic acid | 0.05 |
| Biotin | 0.001 |
| Folic acid | 0.025 |
| Lipoic acid | 0.025 |
| Nicotinic acid | 0.1 |
| Pantothenic acid | 0.05 |
| Pyridoxamin-HCl | 0.25 |
| Vitamin B₁₂ | 0.05 |
| Pyridoxine | 0.025 |
| Riboflavin | 0.05 |
| Thiamine | 0.1 |

Trace element Soln. is disclosed in Kets EPW, Galinski EA, de Bont JAM. Carnitine: "A novel compatible solute in Lactobacillus plantarum", Arch. Microbiol., 192, 243-248 (1994), and the composition is as follows:

| Trace element Soln. | [g/L] |
|---|---|
| 25% HCl | 10 mL |
| FeCl₂ • 4H₂O | 1.5 |
| CoCl₂ • 6H₂O | 0.19 |
| MnCl₂ • 4H₂O | 0.1 |
| ZnCl₂ | 0.07 |
| H₃BO₃ | 0.006 |
| Na₂MoO₄ • 2H₂O | 0.036 |
| NiCl₂ • 6H₂O | 0.024 |
| CuCl₂ • 2H₂O | 0.002 |

Phenol sulfuric acid method (DuBois M, Gilles KA, Hamilton JK, Rebers PA, Smith F., "Colorimetric method for determination of sugars and related substances", Anal. Chem., 28, 350-356 (1956))

30 µL of a subject sample was mixed with an equal amount of 5 w/v% phenol aqueous solution, and 150 µL of a concentrated sulfuric acid was added to the resultant mixture and mixed with each other to allow a reaction to start. Immediately after 10 minutes, the reaction solution was cooled by ice to stop the reaction. The concentration of saccharides was obtained by measuring the absorbance of the reaction solution at 490 nm. The concentration was determined using a calibration curve prepared by performing the same experiment using glucose as a standard.

### 2. Structural analysis of neutral exopolysaccharide

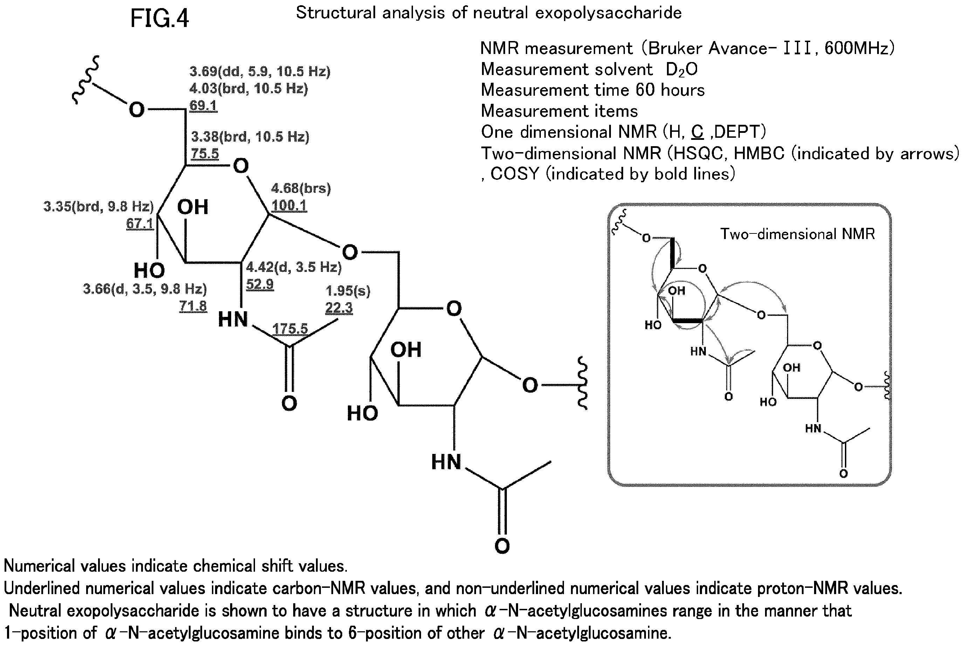

The neutral exopolysaccharide purified by the aforementioned anion exchange column chromatography (TOYOPEARL DEAE-650 M resin (Tosoh Corporation)) was subjected to proton-NMR and carbon-NMR, and the obtained NMR profiles are each illustrated in Fig. 3. The structural analysis results of the neutral exopolysaccharide from these NMR profiles are illustrated in Fig. 4.

From the structural analysis results, it was revealed that the neutral exopolysaccharide produced by the strain IJH-SONE68 has a structure in which N-acetylglucosamines are linked with each other via α-1,6 bond.

### 3. Saccharide composition analysis of acidic exopolysaccharide

The saccharide composition analysis of the aforementioned acidic exopolysaccharide purified by the anion exchange column chromatography was performed by measuring the composition by a high performance liquid chromatography (HPLC) method.

A 7-fold diluted sample solution was prepared by mixing 10 µL of the purified acidic exopolysaccharide (7.3 mg/mL) and 60 µL of water and placed in a test tube. 20 µL of the diluted sample solution was collected from the test tube, dried under reduced pressure, and 100 µL of 2 mol/L trifluoroacetic acid was added thereto to dissolve the dried sample. The resultant solution was substituted with nitrogen, sealed under a reduced pressure, hydrolyzed at 100°C for 6 hours, and then dried under a reduced pressure. To the obtained residue, 200 µL of water was added, dissolved, and filtrated with 0.22 µm filter, to obtain a sample solution for measurement. The sample solution for measurement was 10-fold diluted with water to obtain a sample solution for dilution measurement. 50 µL of each of these sample solutions was analyzed. HPLC system: LC-20A system (Shimadzu Corporation) and spectrofluorophotometer M-10AxL (Shimadzu Corporation) were used as analytical instruments. The analysis conditions were as follows:
Column: TSK-gel Sugar AXG 4.6 mml. D. × 15 cm (Tosoh Corporation)
Column temperature: 70°C
Mobile phase: 0.5 mol/L potassium borate buffer, pH 8.7
Mobile phase flow rate: 0.4 mL/min
Post column labeling: reaction reagent: 1 w/v% arginine• 3 w/v% boric acid
Reaction reagent flow rate: 0.5 mL/min
Reaction temperature: 150°C
Detection wavelength: Ex. 320 nm, Em. 430 nm

Chromatograms of samples prepared from the acidic exopolysaccharide and calibration curve data of each monosaccharide were obtained. The concentrations of constituent saccharides of the acidic exopolysaccharide in the samples were determined from calibration curves. The obtained results are shown in Table 2.

**[Table 2]**

| Constituent saccharides of acidic exopolysaccharide | | |
|---|---|---|
| Acidic exopolysaccharide | | Concentration in sample (mg/mL) |
| Monosaccharides | Rhamnose | 0.0204 |
| | Ribose | n.d. |
| | Mannose | 3.43 |
| | Arabinose | n.d. |
| | Galactose | 0.0384 |
| | Xylose | n.d. |
| | Glucose | 0.219 |

| | | |
|---|---|---|
| In the Table, n.d. indicates no detection. | | |

### Example 3

### Fat accumulation-suppressing action of polysaccharides produced by the strain IJH-SONE68

The fat accumulation-suppressing action of the polysaccharide sample containing the neutral polysaccharide fractions and the acidic polysaccharide fractions, which were exopolysaccharides produced by the strain IJH-SONE68 and obtained in Example 2, was investigated according to the method disclosed below.

### 1. Test method

Human liver cancer-derived cell line, HuH-7 cells, were used as test subjects. HuH-7 cells were cultured in DMEM medium (Dulbecco's modified Eagle medium (containing high glucose, L-glutamine, phenol red, and sodium pyruvate)) containing 10 v/v% FBS (fetal bovine serum), 100 U/mL penicillin G, and 100 µg/mL streptomycin. The resultant confluent cells were suspended in a new DMEM medium and seeded on a 24-well cell culture plate at 8 × 10⁴ cells/well. The liquid volume per well at that time was 500 µL. After culturing for 24 hours, a solution of palmitic acid (PA) and oleic acid (OA) (PA/OA solution) (= 1:2, dissolved in DMSO)) was added to each well at a final concentration of 600 µM (PA: 200 µM and OA: 400 µM)), and further cultured for 24 hours to start intake of fatty acids. At that time, each polysaccharide sample (final concentration: 4-400 µg/mL) was added at the same time, and the suppression on the intake of fatty acids into cells was evaluated.

After 24 hours, the culture solution in each well was removed with an aspirator, and 500 µL of PBS (phosphate buffered saline) was added thereto to wash the cells and the wells. After removing the PBS, 250 µL of 10 w/v% formalin solution was added, and the cells were immobilized for 10 minutes. Subsequently, the wells were washed twice with 500 µL of PBS, and after removing the PBS, 150 µL of a color former solution (triglyceride E-Test Wako) was added, and a color reaction was performed at 37°C for 30 minutes. After the reaction, 100 µL of the reaction solution was recovered from each well, and the absorbance at 600 nm was measured to determine and compare the amount of fatty acid incorporated into the cells as the amount of intracellular triglyceride (neutral fat). A polysaccharide produced by *Lactobacillus plantarum* strain SN35N (Accession No. NITE P-6) was used as a control.

### 2. Test results

The evaluation of fatty acid intake was performed twice (1st and 2nd), and the obtained results are shown in Table 3.

**[Table 3]**

| Accumulation amount of neutral fat in HuH-7 cells due to the addition of various EPS | | | | | |
|---|---|---|---|---|---|
| Polysaccharide sample | Final concentration of polysaccharide (µg/mL) | Accumulation amount of neutral fat (A_{600 nm}) | | | |
| | | 1st | 2nd | Average | SE |
| NC | - | 0.107 | 0.098 | 0.102 | 0.004 |
| Vehicle | - | 0.104 | 0.095 | 0.100 | 0.004 |
| PA/OA | - | 0.347 | 0.360 | 0.353 | 0.007 |
| SONE68 | 4 | 0.198 | 0.279 | 0.239 | 0.041 |
| SN35N | 4 | 0.201 | 0.313 | 0.257 | 0.056 |
| SONE68 | 40 | 0.098 | 0.222 | 0.160 | 0.062 |
| SN35N | 40 | 0.188 | 0.272 | 0.230 | 0.042 |
| SONE68 | 400 | 0.085 | 0.103 | 0.094 | 0.009 |
| SN35N | 400 | 0.122 | 0.133 | 0.128 | 0.006 |

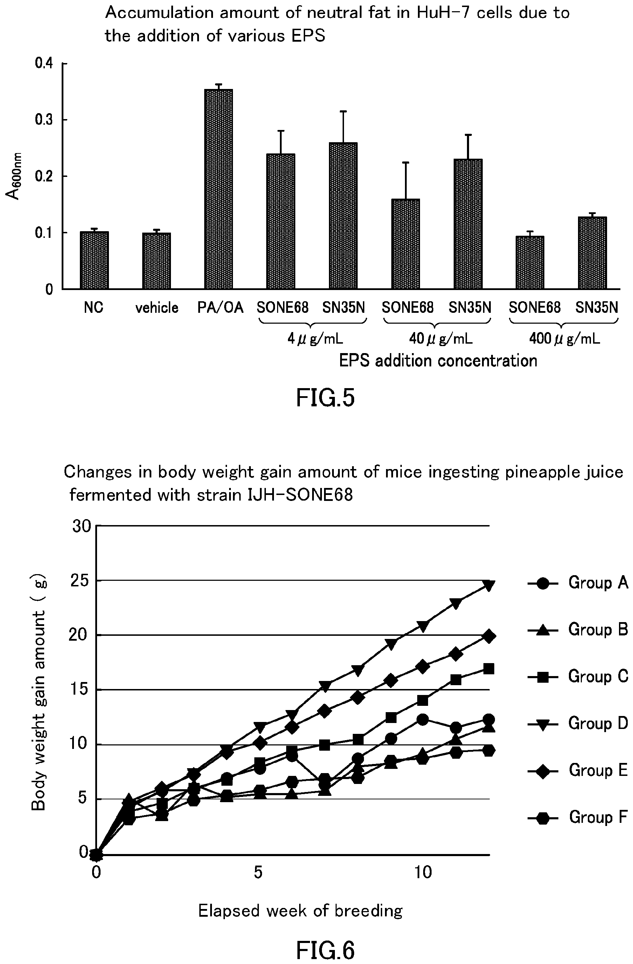

In addition, Fig. 5 also shows the obtained results. As can be seen from the results in Table 3 and Fig. 5, the addition of only the PA/OA solution clearly increased the triglyceride (neutral fat) content in the cells. In addition, when only DMSO (vehicle) as a solvent, in which the PA/OA was dissolved, was added, no change was observed like the case where nothing was added (NC). On the other hand, when a polysaccharide sample (EPS) (SONE68) produced by the strain IJH-SONE68 was added in the presence of the PA/OA, it was observed that the intake of fatty acids into cells was suppressed in a concentration-dependent manner and that fat accumulation was suppressed. The degree of the suppression was higher in the polysaccharide sample produced by the strain IJH-SONE68 than in the polysaccharide sample (EPS) (SN35N) produced by the strain SN35N.

From the above results, it was revealed that the polysaccharide produced by the strain IJH-SONE68 has an action of suppressing fat accumulation.

### Example 4

### Anti-obesity action and fat accumulation-suppressing action of the strain JIH-SONE68

The anti-obesity action and fat accumulation-suppressing action of the strain IJH-SONE68 were evaluated using obese model mice ingesting high fat diets. As a result, it was revealed that the ingestion of a fermented pineapple juice fermented with the strain IJH-SONE68 significantly suppressed body weight gain and visceral fat accumulation in obese model mice ingesting high fat diets.

### 1. Test method

In the present test, C57BL/6Jcl (SPF) male mice were used. Seven-week-old mice were brought in a breeding cage, and five mice were kept per the breeding cage. After one week of acclimatization using normal diets (MF, manufactured by Oriental Yeast Co., Ltd.), the mice were divided into groups of 5 mice (Groups A to E), and the diets were changed to high fat diets (Research Diet, # D12492), and the induction of obesity was started. When feeding the diets, the high-fat diets were ground in advance with a mallet to make them clay-like and packed in a powder feeder made of glass (KN-675-4A) so that 120 g was set per the feeding. The changes were made at weekly intervals. In addition, the administration sample was mixed with the high-fat diets so as to be uniform before packing in the feeder. All groups had free access to the diets and drinking water. The undiluted solution of a pineapple juice fermented with the strain IJH-SONE68, which was obtained by sterilizing a fermented broth obtained by culturing the strain IJH-SONE68 in 100% pineapple juice (containing 1 w/v% distillation residue of distilled spirit) for 48 hours, at 121°C for 20 minutes, and its diluted solution were used as administration samples. The details of the administration samples for Groups A to F of the mice were as follows.

Group A: a group in which the mice ingested an undiluted solution of a pineapple juice fermented with the strain IJH-SONE68, together with high-fat diets (a group in which the mice ingested the mixture of 7 mL of an undiluted solution of the fermented pineapple juice and 120 g of high-fat diets).

Group B: a group in which the mice ingested a 10-fold diluted solution of a pineapple juice fermented with the strain IJH-SONE68, together with high-fat diets (a group in which the mice ingested the mixture of 7 mL of a 10-fold diluted fermented pineapple juice diluted with sterile distilled water and 120 g of high-fat diets).

Group C: a group in which the mice ingested a 100-fold diluted solution of a pineapple juice fermented with the strain IJH-SONE68, together with high-fat diets (a group in which the mice ingested the mixture of 7 mL of a 100-foled diluted solution of the fermented pineapple juice diluted with sterile distilled water and 120 g of high-fat diets).

Group D: a group in which the mice ingested an unfermented pineapple juice together with high-fat diets (a group in which the mice ingested the mixture of 7 mL of the pineapple juice and 120 g of high-fat diets).

Group E: a group in which the mice were bred only with high-fat diets (a positive control group) (a group in which the mice ingested the mixture of 7 mL of sterile distilled water and 120 g of high-fat diets).

Group F: a group in which the mice were bred only with an ordinary feed (a negative control group).

After the content of the diets was changed from the high-fat diets to each of the above-mentioned diets of Groups A to F, the breeding was continued for 12 weeks. The body weight of the mice was measured every week during the breeding period. After the end of the breeding period, the mice were euthanized by isoflurane inhalation anesthesia, visceral fat was collected, and its weight was measured. An independent t-test was used for a significant difference test in each measurement data, and when a risk rate (p value) was less than 0.05, it was determined that there was a significant difference.

### 2. Test results and discussion

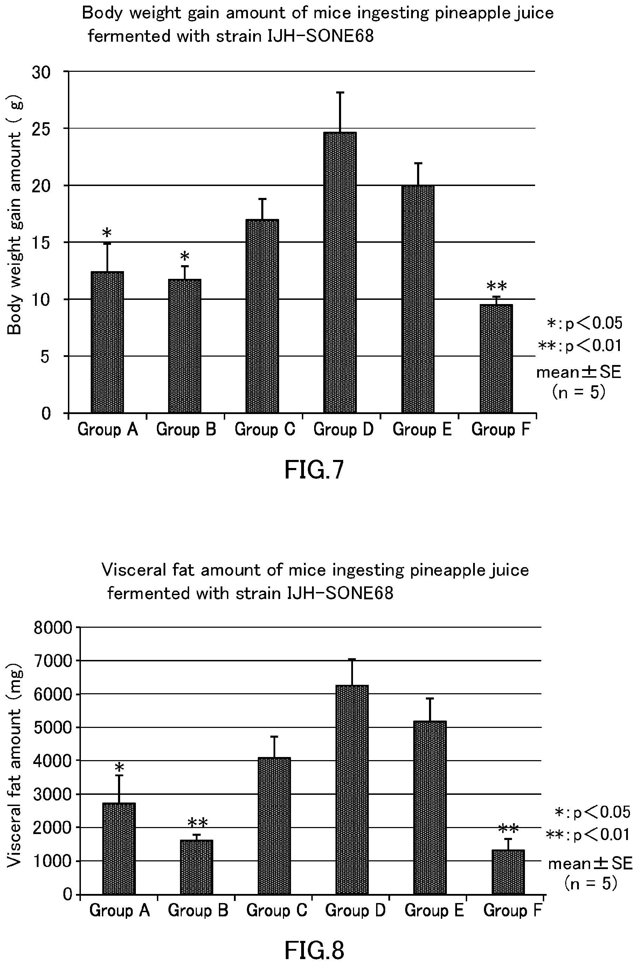

Tables 4 and 5 show changes in the amount of body weight gain every one week (the amount increased from body weight at the start of breeding (week 0)) in each of Groups A to F (Table 4 shows the average body weight gain amount (g), and Table 5 shows the standard errors (SE)), and graphs thereof are shown in Fig. 6. In addition, Table 6 shows the average body weight gain amount of each Group at the last week of breeding (week 12), and graphs thereof are shown in Fig. 7. Each of the data is shown as a mean ± standard error, and Tukey-Kramer method was used for a significant difference test among the Groups, and it was determined that there was a significant difference when a risk factor (p value) was less than 0.05 (Figs. 6 and 7 disclosed herein below show only a significant difference from the positive control group).

**[Table 4]**

| Average value (g) of body weight gain amount every elapsed week in each feed administration group (n = 5 for each group) | | | | | | |
|---|---|---|---|---|---|---|
| Elapsed weeks | Group A | Group B | Group C | Group D | Group E | Group F |
| 0 | 0 | 0 | 0 | 0 | 0 | 0 |
| 1 | 4.26 | 5.12 | 3.88 | 4.60 | 4.82 | 3.27 |
| 2 | 5.82 | 3.48 | 4.66 | 5.64 | 6.02 | 3.73 |
| 3 | 5.84 | 6.32 | 6.04 | 7.50 | 7.32 | 5.00 |
| 4 | 6.98 | 5.24 | 6.88 | 9.62 | 9.38 | 5.40 |
| 5 | 7.94 | 5.52 | 8.38 | 11.62 | 10.22 | 5.90 |
| 6 | 9.06 | 5.52 | 9.44 | 12.78 | 11.64 | 6.63 |
| 7 | 6.38 | 5.78 | 9.98 | 15.46 | 13.10 | 6.90 |
| 8 | 8.78 | 8.02 | 10.52 | 16.88 | 14.38 | 7.07 |
| 9 | 10.58 | 8.26 | 12.58 | 19.32 | 15.92 | 8.60 |
| 10 | 12.32 | 9.16 | 14.06 | 20.92 | 17.18 | 8.80 |
| 11 | 11.52 | 10.54 | 15.98 | 22.98 | 18.28 | 9.33 |
| 12 | 12.34 | 11.68 | 16.94 | 24.62 | 19.98 | 9.50 |

**[Table 5]**

| Standard error (SE) of average value (g) of body weight gain amount every elapsed week in each feed administration group (n = 5 for each group) | | | | | | |
|---|---|---|---|---|---|---|
| Elapsed weeks | Group A | Group B | Group C | Group D | Group E | Group F |
| 0 | 0 | 0 | 0 | 0 | 0 | 0 |
| 1 | 0.69 | 1.06 | 0.23 | 1.61 | 1.20 | 1.16 |
| 2 | 0.59 | 0.84 | 0.37 | 1.77 | 1.21 | 1.42 |
| 3 | 1.11 | 1.03 | 0.40 | 1.97 | 1.15 | 1.57 |
| 4 | 1.15 | 0.69 | 0.62 | 2.23 | 1.16 | 1.80 |
| 5 | 1.49 | 1.07 | 0.82 | 2.36 | 1.11 | 1.67 |
| 6 | 1.60 | 0.73 | 0.97 | 2.69 | 1.26 | 1.20 |
| 7 | 1.08 | 0.80 | 1.16 | 2.79 | 1.28 | 1.27 |
| 8 | 1.58 | 1.03 | 1.22 | 2.92 | 1.41 | 1.52 |
| 9 | 1.88 | 0.99 | 1.49 | 3.12 | 1.61 | 1.15 |
| 10 | 2.19 | 1.09 | 1.64 | 3.26 | 1.84 | 1.07 |
| 11 | 2.38 | 1.27 | 1.71 | 3.53 | 1.88 | 0.90 |
| 12 | 2.55 | 1.23 | 1.85 | 3.50 | 1.99 | 0.67 |

**[Table 6]**

| Average value (g) and standard error (SE) of body weight gain amount after 12 weeks in each feed administration group (n = 5 for each group) | | | | | | |
|---|---|---|---|---|---|---|
| Week 12 | Group A | Group B | Group C | Group D | Group E | Group F |
| Average value | 12.34 | 11.68 | 16.94 | 24.62 | 19.98 | 9.50 |
| SE | 2.55 | 1.23 | 1.85 | 3.50 | 1.99 | 0.67 |

As can be seen from Tables 4 to 6 and Figs. 6 to 7, it was confirmed that the body weight of the positive control group (Group E) was significantly increased by the ingestion of the high-fat diets (p < 0.01), as compared to the negative control group (Group F). It was revealed that among the groups in which the mice ingested the fermented pineapple juice, the group in which the mice ingested the undiluted solution or the 10-fold diluted solution (Group A or B) showed a significant suppression on an increase in body weight due to the ingestion of high-fat diets (p < 0.05 for Groups A and B), as compared to the positive control group. In addition, when the mice ingested the 100-fold diluted solution of the fermented pineapple juice (Group C), the effect was attenuated, but an increase in the body weight was less than that in the positive control group. On the other hand, in the group in which the mice ingested the unfermented pineapple juice (Group D), an increase in the body weight was observed as compared to the positive control group, but no significant difference was observed.

Next, the measurement results of the amount of visceral fat in the mice of each group at the last week of breeding (week 12) are shown in Table 7 and Fig. 8

**[Table 7]**

| Average value (mg) and standard error (SE) of the weight amount of visceral fat in the mice of each group at week 12 of breeding | | | | | | |
|---|---|---|---|---|---|---|
| | Group A | Group B | Group C | Group D | Group E | Group F |
| Average value | 2735 | 1633 | 4108 | 6240 | 5172 | 1346 |
| SE | 828 | 159 | 596 | 789 | 687 | 333 |

As can be seen from Table 7 and Fig. 8, it was confirmed that the visceral fat amount was significantly increased due to the ingestion of the high-fat diets in the positive control group (p < 0.01), as compared to the negative control group. Among the groups in which the mice ingested the fermented pineapple juice, the group in which the mice ingested the 10-fold diluted solution (Group B) showed a significant suppression on an increase in the visceral fat amount (p < 0.01) as compared to the positive control group; however, the ingestion of the undiluted solution (Group A) remained in a suppressive trend (p <0.1). On the other hand, when the mice ingested the 100-fold diluted solution of the fermented pineapple juice (Group C), the effect was attenuated. Furthermore, in the group in which the mice ingested the unfermented pineapple juice (Group D), an increase in the visceral fat amount was observed similarly to the body weight, as compared to the positive control group, but no significant difference was observed.

From the above results, it was revealed that the ingestion of the 10-fold diluted solution of the pineapple juice fermented with the strain IJH-SONE68 significantly suppressed both an increase in the body weight and an increase in the visceral fat, induced by the ingestion of high-fat diets. In the case of the ingestion of the undiluted solution and 100-fold diluted solution of the pineapple juice fermented with the strain IJH-SONE68, it was confirmed that the effect was attenuated. This is considered to be due to the fact that components involved in the functionality of the mice were diluted in the 100-fold diluted solution. When the mice ingested the unfermented pineapple juice, the body weight increased more than the positive control group. Therefore, in the case of the pineapple juice fermented with the strain IJH-SONE68, it was considered that there was a possibility that the increase in the body weight was due to sugars remaining in the pineapple juice.

As is clear from the foregoing detailed disclosures, the present invention provides the following inventions:
[1] A composition for suppressing fat accumulation, comprising, as an active ingredient, a cell body of a lactic acid bacterium belonging to *Lactobacillus paracasei,* a cultured product or fermented product thereof, or a polysaccharide produced thereby.
[2] The composition according to the above [1], wherein the composition is for preventing or improving obesity.
[3] The composition according to the above [1], wherein the composition is for preventing or improving fatty liver or liver dysfunction.
[4] The composition according to any one of the above [1] to [3], wherein the lactic acid bacterium is a lactic acid bacterium derived from a fig.
[5] The composition according to any one of the above [1] to [4], wherein the lactic acid bacterium is *Lactobacillus paracasei* strain IJH-SONE68 (Accession No. NITE BP-02242) or a lactic acid bacterium equivalent thereto.
[6] The composition according to any one of the above [1] to [5], wherein the cultured product or fermented product of the lactic acid bacterium is a cultured product or fermented product that is obtained by cultivating or fermenting the lactic acid bacteria in the presence of a fruit juice of pineapple genus plant.
[7] The composition according to any one of the above [1] to [5], wherein the polysaccharide is a neutral polysaccharide having a structure in which N-acetylglucosamines are linked with each other via α-1,6 bond.
[8] The composition according to any one of the above [1] to [5], wherein the polysaccharide is an acidic polysaccharide composed mainly of glucoses and mannoses.
[9] The composition according to any one of the above [1] to [8], wherein the composition is a food or drink composition.
[10] The composition according to the above [9], wherein the food or drink is a beverage, a functional food, a fermented food, or a supplement.
[11] The composition according to any one of the above [1] to [8], wherein the composition is a pharmaceutical composition.
[12] The composition according to any one of the above [1] to [8], wherein the composition is a feed composition.
[13] Use of a cell body of a lactic acid bacterium belonging to *Lactobacillus paracasei,* a cultured product or fermented product thereof, or a polysaccharide produced thereby, as an active ingredient of a composition for suppressing fat accumulation.
[14] The use according to the above [13], wherein the composition is for preventing or improving obesity.
[15] The use according to the above [13], wherein the composition is for preventing or improving fatty liver or liver dysfunction.
[16] The use according to any one of the above [13] to [15], wherein the lactic acid bacterium is a lactic acid bacterium derived from a fig.
[17] The use according to any one of the above [13] to [16], wherein the lactic acid bacterium is *Lactobacillus paracasei* strain IJH-SONE68 (Accession No. NITE BP-02242) or a lactic acid bacterium equivalent thereto.
[18] The use according to any one of the above [13] to [17], wherein the cultured product or fermented product of the lactic acid bacterium is a cultured product or fermented product that is obtained by cultivating or fermenting the lactic acid bacteria in the presence of a fruit juice of pineapple genus plant.
[19] The use according to any one of the above [13] to [17], wherein the polysaccharide is a neutral polysaccharide having a structure in which N-acetylglucosamines are linked with each other via α-1,6 bond.
[20] The use according to any one of the above [13] to [17], wherein the polysaccharide is an acidic polysaccharide composed mainly of glucoses and mannoses.
[21] The use according to any one of the above [13] to [20], wherein the composition is a food or drink composition.
[22] The use according to the above [21], wherein the food or drink is a beverage, a functional food, a fermented food, or a supplement.
[23] The use according to any one of the above [13] to [20], wherein the composition is a pharmaceutical composition.
[24] The use according to any one of the above [13] to [20], wherein the composition is a feed composition.
[25] A method for applying a composition comprising, as an active ingredient, a cell body of a lactic acid bacterium belonging to *Lactobacillus paracasei,* a cultured product or fermented product thereof, or a polysaccharide produced thereby, to a subject in need thereof, wherein the method suppresses fat accumulation in the subject.
[26] The method according to the above [25], wherein the method prevents or improves obesity.
[27] The method according to the above [25], wherein the method prevents or improves fatty liver or liver dysfunction.
[28] The method according to any one of the above [25] to [27], wherein the lactic acid bacterium is a lactic acid bacterium derived from a fig.
[29] The method according to any one of the above [25] to [28], wherein the lactic acid bacterium is *Lactobacillus paracasei* strain IJH-SONE68 (Accession No. NITE BP-02242) or a lactic acid bacterium equivalent thereto.
[30] The method according to any one of the above [25] to [29], wherein the cultured product or fermented product of the lactic acid bacterium is a cultured product or fermented product that is obtained by cultivating or fermenting the lactic acid bacteria in the presence of a fruit juice of pineapple genus plant.
[31] The method according to any one of the above [25] to [30], wherein the polysaccharide is a neutral polysaccharide having a structure in which N-acetylglucosamines are linked with each other via α-1,6 bond.
[32] The method according to any one of the above [25] to [30], wherein the polysaccharide is an acidic polysaccharide composed mainly of glucoses and mannoses.
[33] The method according to any one of the above [25] to [32], wherein the composition is a food or drink composition.
[34] The method according to the above [33], wherein the food or drink is a beverage, a functional food, a fermented food, or a supplement.
[35] The method according to any one of the above [25] to [32], wherein the composition is a pharmaceutical composition.
[36] The method according to any one of the above [25] to [32], wherein the composition is a feed composition.

### Industrial Applicability

As disclosed in detail herein above, the composition of the present invention is effective in suppressing fat accumulation and can be used to prevent or improve obesity and also prevent or improve fatty liver or liver dysfunction. The composition of the present invention can be used as a food or drink, a medicine, and a feed. The composition of the present invention comprises, as an active ingredient, a cell body of a lactic acid bacterium belonging to *Lactobacillus paracasei,* a cultured product or fermented product thereof, or a polysaccharide produced thereby. Therefore, the composition of the present invention has high safety, can be applied for a long period of time, can be supplied in a large amount at low cost, and has extremely high utility and practicality.

## Claims

1. A composition for suppressing fat accumulation, comprising, as an active ingredient, a cell body of a lactic acid bacterium belonging to *Lactobacillus paracasei,* a cultured product or fermented product thereof, or a polysaccharide produced thereby.

2. The composition according to claim 1, wherein the composition is for preventing or improving obesity.

3. The composition according to claim 1, wherein the composition is for preventing or improving fatty liver or liver dysfunction.

4. The composition according to any one of claims 1 to 3, wherein the lactic acid bacterium is a lactic acid bacterium derived from a fig.

5. The composition according to any one of claims 1 to 4, wherein the lactic acid bacterium is *Lactobacillus paracasei* strain IJH-SONE68 (Accession No. NITE BP-02242) or a lactic acid bacterium equivalent thereto.

6. The composition according to any one of claims 1 to 5, wherein the cultured product or fermented product of the lactic acid bacterium is a cultured product or fermented product that is obtained by cultivating or fermenting the lactic acid bacteria in the presence of a fruit juice of pineapple genus plant.

7. The composition according to claims 1 to 5, wherein the polysaccharide is a neutral polysaccharide having a structure in which N-acetylglucosamines are linked with each other via α-1,6 bond.

8. The composition according to any one of claims 1 to 6, wherein the polysaccharide is an acidic polysaccharide composed mainly of glucoses and mannoses.

9. The composition according to any one of claims 1 to 8, wherein the composition is a food or drink composition.

10. The composition according to claim 9, wherein the food or drink is a beverage, a functional food, a fermented food, or a supplement.

11. The composition according to any one of claims 1 to 8, wherein the composition is a pharmaceutical composition.

12. The composition according to any one of claims 1 to 8, wherein the composition is a feed composition.
